# EUROPEAN PATENT SPECIFICATION

(11) **EP 3 875 135 B1**
(45) Date of publication and mention of the grant of the patent: **23.08.2023**
(21) Application number: 18938519.8
(22) Date of filing: 31.10.2018
(51) Int. Cl.: A61M 16/01, A61M 16/08

(54) **ANESTHESIA RESPIRATION DEVICE**
ANÄSTHESIEBEATMUNGSVORRICHTUNG
DISPOSITIF DE RESPIRATION D'ANESTHÉSIE

(43) Date of publication of application: 08.09.2021
(73) Proprietor: Shenzhen Mindray Bio-Medical Electronics Co., Ltd., Shenzhen, Guangdong 518057 (CN); Shenzhen Mindray Scientific Co., Ltd., Shenzhen, Guangdong 518057 (CN)
(72) Inventor: CHEN, Peitao, Shenzhen, Guangdong 518057 (CN); CAI, Kun, Shenzhen, Guangdong 518057 (CN); LUO, Caijin, Shenzhen, Guangdong 518057 (CN); YUAN, Sheng, Shenzhen, Guangdong 518057 (CN)
(74) Representative: KIPA AB
(86) International application number: PCT/CN2018/113182
(87) International publication number: WO 2020/087397

(56) References cited:
- EP-A1- 2 201 979
- WO-A1-2009/062547
- WO-A1-2016/086347
- CN-A- 103 071 219
- US-A1- 2010 078 018
- US-A1- 2010 252 046
- US-A1- 2013 061 852
- US-A1- 2015 114 395
- US-A1- 2015 374 947
- US-B1- 6 892 726

## Description

### TECHNICAL FIELD

Embodiments of the disclosure relate to the technical field of medical instruments, and in particular to an anesthesia respiration device and method.

### BACKGROUND

As a medical apparatus that can provide oxygen, anesthesia and respiratory support, anesthesia machines have been commonly used during patients' operations. However, the existing pneumatic anesthesia machine can work only with an external driving gas source, which limits the use of the anesthesia machine to a certain extent.

Some existing techniques, e.g., US20150374947A1, US20150114395A1, EP2201979A1, US20130061852A1, US20100078018A1, WO2016086347A1, CN103071219A, US0100252046A1, WO2009062547A1 and US6892726B1, discloses a reflecting component used in the anesthesia and respiratory machine. The drive gas provided from external driving gas source can pass through the reflecting component, and drive the inhaled gas to the patient. In the expiration phase, the patient-exhaled gas can enter the reflecting component and the drive gas inside the reflecting component can be discharged by the evacuation line of the anesthesia and respiratory machine.

### SUMMARY

In order to solve the above technical problem, the embodiments of the disclosure expect to provide an anesthesia respiration device method, which can work without an external driving gas source.

The technical solutions of the embodiments of the disclosure may be implemented as follows:
an embodiment of the disclosure provides an anesthesia respiration device, which includes:
   a fresh gas input for inputting a fresh gas;
   an internal loop circuit for storing a patient-exhaled gas and providing the fresh gas and the patient-exhaled gas to the patient; the internal loop circuit comprises an expiratory branch for receiving the patient-exhaled gas through a patient interface; a reflecting component for storing the patient-exhaled gas; an inspiratory branch for transferring the fresh gas and the patient-exhaled gas that is stored in the reflecting component to the patient through the patient interface; the inspiratory branch is connected to the reflecting component and the fresh gas input; an expiratory pressure control branch for controlling a pressure of the patient-exhaled gas, the expiratory pressure control branch is connected to the reflecting component; and
an external control branch for controlling the internal loop circuit; wherein the external control branch comprises a drive branch, the drive branch comprises:
   a pressurizing component for controlling transmission of the patient-exhaled gas in the internal loop circuit to the patient; and wherein
   the reflecting component is connected to the expiratory branch and the drive branch, and the fresh gas input and the external control branch are separately connected to the internal loop circuit;
   the external control branch further includes: a pressure-controlled gas supply branch, connected to the expiratory pressure control branch, for providing a pressure-controlled gas, and controlling the expiratory pressure control branch to perform pressure control on the patient-exhaled gas.

In the above device, the pressurizing component is any one of a turbine, a pressurizing pump, an air compressor and a cylinder.

In the above device, the drive branch further comprises: at least one of a filter and a flow sensor; wherein
an outlet of the filter is connected to the pressurizing component; and
the flow sensor is connected between the pressurizing component and the internal loop circuit.

In the above device, the pressure-controlled gas supply branch comprises:
a pressure control component connected to the expiratory pressure control branch.

In the above device, the pressure control component is any one of a turbine, a pressurizing pump, an air compressor and a cylinder.

In the above device, the pressure-controlled gas supply branch comprises: a gas source interface and an electromagnetic proportional valve which are connected in sequence;
wherein an outlet of the electromagnetic proportional valve is connected to the expiratory pressure control branch.

In the above device,
the pressure-controlled gas supply branch is connected between the pressurizing component and the expiratory pressure control branch.

In the above device, the pressure-controlled gas supply branch comprises:
a pressure sensor connected between the pressurizing component and the expiratory pressure control branch.

In the above device, the drive branch further comprises:
a switch valve connected between the pressurizing component and the reflecting component.

In the above device, the expiratory pressure control branch comprises: an expiratory valve and a gas resistor;
wherein an inlet of the expiratory valve is connected to the reflecting component; and
the gas resistor is connected to a valve gate of the expiratory valve.

It can be seen therefrom that the anesthesia respiration device of an embodiment of the disclosure comprises: a fresh gas input for inputting a fresh gas; an internal loop circuit for storing a patient-exhaled gas and providing the fresh gas and the patient-exhaled gas to the patient; and an external control branch for controlling the internal loop circuit; wherein the external control branch comprises a drive branch, which comprises: a pressurizing component for controlling transmission of the patient-exhaled gas in the internal loop circuit to the patient; and the fresh gas input and the external control branch are separately connected to the internal loop circuit. That is to say, in the anesthesia respiration device provided by the embodiment of the disclosure, the pressurizing component does not in direct contact with the patient-exhaled gas and other impurities, so as to ensure the working capability and the accuracy of the pressurizing component.

### BRIEF DESCRIPTION OF THE DRAWINGS

FIG. 1 is schematic structural diagram I of an anesthesia respiration device provided by an embodiment of the disclosure;
FIG. 2 is schematic structural diagram II of an anesthesia respiration device provided by an embodiment of the disclosure;
FIG. 3 is schematic structural diagram III of an anesthesia respiration device provided by an embodiment of the disclosure.

### DESCRIPTION OF THE PREFERRED EMBODIMENTS

In order to understand the features and technical contents of embodiments of the disclosure in more detail, the implementation of the embodiments of the disclosure will be described below in detail with reference to the accompanying drawings, which are for reference and illustration only, and are not intended to limit the embodiments of the disclosure.

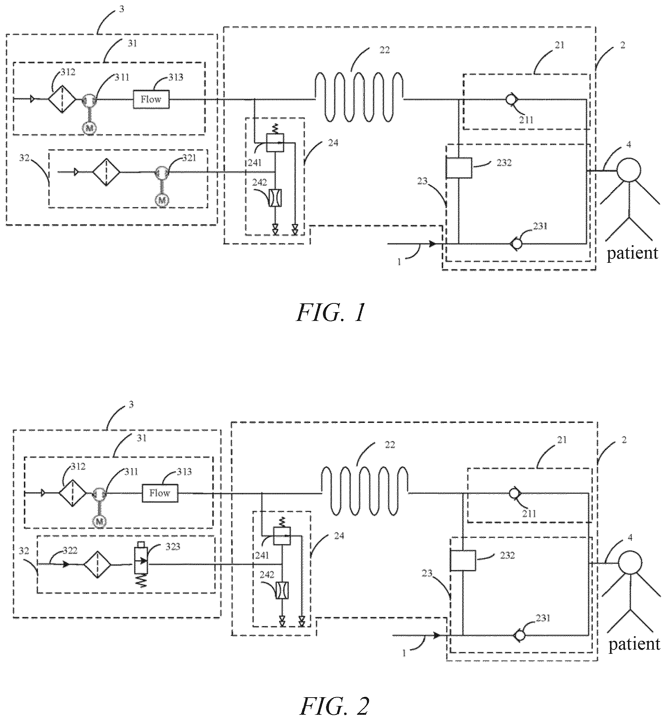

An embodiment of the disclosure provides an anesthesia respiration device, with a schematic structural diagram thereof as shown in FIG. 1. The anesthesia respiration device comprises:
a fresh gas input 1 for inputting a fresh gas;
an internal loop circuit 2 for storing a patient-exhaled gas and providing the fresh gas and the patient-exhaled gas to the patient; and
an external control branch 3 for controlling the internal loop circuit 2. The external control branch 3 comprises a drive branch 31. The drive branch 31 comprises:
   a pressurizing component 311 for controlling transmission of the patient-exhaled gas in the internal loop circuit 2 to the patient.

The fresh gas input 1 and the external control branch 3 are separately connected to the internal loop circuit 2.

In the embodiment of the disclosure, the pressurizing component 311 for controlling transmission of the patient-exhaled gas in the internal loop circuit 2 to the patient is disposed outside the internal loop circuit 2, and can effectively prevent the pressurizing component 311 from direct contact with the patient-exhaled gas and other impurities, so as to ensure the working capability and accuracy of the pressurizing component 311.

It should be noted that in the embodiment of the disclosure, the fresh gas that is input through the fresh gas input 1 may be a mixed gas of oxygen and anesthetic gas. The specific fresh gas is not limited in the embodiment of the disclosure.

Specifically, in the embodiment of the disclosure, the pressurizing component 311 may be any one of a turbine, a pressurizing pump, an air compressor and a cylinder, and the specific pressurizing component 311 is not limited in the embodiment of the disclosure.

It should be noted that in the embodiment of the disclosure, the pressurizing component 311 may generate a driving gas. For example, the pressurizing component 311 is a turbine. During an inspiratory stage, the turbine can suck the gas in the atmosphere so as to generate the driving gas. When the driving gas is transferred to the internal loop circuit 2, the patient-exhaled gas stored in the internal loop circuit 2 and the fresh gas that is input to the internal loop circuit 2 through the fresh gas input 1 can be drived together to the patient.

Specifically, in the embodiment of the disclosure, the drive branch 31 may further comprise: a filter 312 and/or a flow sensor 313.

An outlet of the filter 312 is connected to the pressurizing component 311.

The flow sensor 313 is connected between the pressurizing component 311 and the internal loop circuit 2, and can measure the flow.

It can be understood that in the embodiment of the disclosure, before the gas in the atmosphere is sucked by the pressurizing component 311 to generate a driving gas, the gas can firstly be subjected to certain filtration by means of the filter 312, avoiding the contamination to the pressurizing component 311. The flow sensor 313 can detect the flow of the gas output by the pressurizing component 311 in real time, which is convenient for real-time adjustment of the output of the pressurizing component 311. For example, the pressurizing component 311 is a turbine, and the rotation speed of the turbine can be adjusted so as to adjust the flow of the output driving gas.

Specifically, in the embodiment of the disclosure, the internal loop circuit 2 comprises:
an expiratory branch 21 for receiving the patient-exhaled gas through a patient interface 4;
a reflecting component 22 connected to the expiratory branch 21 and the drive branch 31 and storing the patient-exhaled gas;
an inspiratory branch 23 connected to the reflecting component 22 and the fresh gas input 1 and transferring the fresh gas and the patient-exhaled gas that is stored in the reflecting component 22 to the patient through the patient interface 4; and
an expiratory pressure control branch 24 connected to the reflecting component 22 and controlling the pressure of the patient-exhaled gas.

It should be noted that in the embodiment of the disclosure, the reflecting component 22 may be any one of a volume reflector and a folding balloon. The specific reflecting component 22 is not limited in the embodiment of the disclosure.

It can be understood that in the embodiment of the disclosure, since the volume reflector is composed of a small pipe, it can effectively isolate the driving gas and the patient-exhaled gas to prevent the loss of an anesthetic in the patient-exhaled gas.

It should be noted that in the embodiment of the disclosure, the expiratory branch 21 comprises an expiratory one-way valve 211, and the inspiratory branch 23 comprises an inspiratory one-way valve 231 and an absorption component 232 for absorbing carbon dioxide. An inlet of the reflecting component 22 is connected to the drive branch 31, an outlet thereof is connected to the expiratory one-way valve 211 and the absorption component 232, and the inspiratory one-way valve 231 is connected to the fresh gas input 1 and the absorption component 232. Of course, the absorption component 232 may also be disposed between the fresh gas input 1 and the inspiratory one-way valve 231.

It should be noted that in the embodiment of the disclosure, during the inspiratory stage, the pressurizing component 311 generates and transfers a driving gas to the reflecting component in the internal loop circuit 2 to drive the patient-exhaled gas stored in the reflecting component 22 to the inspiratory branch 23; and when the patient-exhaled gas flows through the absorption component 232, the absorption component 232 can absorb the carbon dioxide in the patient-exhaled gas and output rebreathing gas that can be inhaled again by the patient, and the rebreathing gas and the fresh gas that is input through the fresh gas input 1 are transferred to the patient interface 4 through the inspiratory one-way valve 231 and are finally transferred to the patient through the patient interface 4.

It should be noted that in the embodiment of the disclosure, during an expiratory stage, the patient exhales the patient-exhaled gas, and the patient-exhaled gas is transferred to the reflecting component 22 through the expiratory one-way valve 211.

Specifically, in the embodiment of the disclosure, the expiratory pressure control branch 24 comprises: an expiratory valve 241 and a gas resistor 242.

An inlet of the expiratory valve 241 is connected to the reflecting component 22.

The gas resistor 242 is connected to a valve gate of the expiratory valve 241.

During the expiratory stage, the patient-exhaled gas pushes the driving gas, input during the inspiratory stage, stored in the reflecting component 22 to move towards the inlet of the reflecting component 22, and the excess driving gas is discharged through the expiratory valve 241.

Specifically, in the embodiment of the disclosure, the external control branch 3 further comprises:
a pressure-controlled gas supply branch 32 connected to the expiratory pressure control branch 24, providing a pressure-controlled gas, and controlling the expiratory pressure control branch 24 to perform control on the pressure of the patient-exhaled gas.

It should be noted that the pressure-controlled gas supply branch 32 can provide and transfer the pressure-controlled gas to the gas resistor 242 of the expiratory pressure control branch 24, and when the pressure-controlled gas flows through the gas resistor 242, the pressure control on the gas output through the expiratory valve 241 can be achieved.

Specifically, in the embodiment of the disclosure, a first type of pressure-controlled gas supply branch 32 comprises:
a pressure control component 321 connected to the expiratory pressure control branch 24.

It should be noted that in the embodiment of the disclosure, similarly, the pressure-controlled gas supply branch 32 can be connected to a filter before the pressure control component 321 to prevent the contamination of the input gas to the internal components, as shown in FIG. 1.

Specifically, in the embodiment of the disclosure, the pressure control component 321 is any one of a turbine, a pressurizing pump, an air compressor and a cylinder, and the specific pressure control component 321 is not limited in the embodiment of the disclosure.

Specifically, in the embodiment of the disclosure, for the first type of pressure-controlled gas supply branch 32, as shown in FIG. 1, the pressurizing component 311 and the pressure control component 321 are both a turbine, the turbine as the pressurizing component 311 can suck the gas in the atmosphere to generate the driving gas, and the turbine as the pressure control component 321 can also suck the gas in the atmosphere to generate the pressure-controlled gas.

Specifically, in another embodiment of the disclosure, a second type of pressure-controlled gas supply branch 32 comprises: a gas source interface 322 and an electromagnetic proportional valve 323 which are connected in sequence.

An outlet of the electromagnetic proportional valve 323 is connected to the expiratory pressure control branch 24.

It should be noted that in the embodiment of the disclosure, the outlet of the electromagnetic proportional valve 323 is specifically connected to the gas resistor 242 of the expiratory pressure control branch 24.

It should be noted that in the embodiment of the disclosure, the gas source interface 322 may be an external medical institution wall end or a gas bottle so as to provide a high-pressure gas source for the electromagnetic proportional valve 323; the electromagnetic proportional valve 323 can control the flow of the input gas so as to generate the pressure-controlled gas that meets actual needs; and in the same way, the pressure-controlled gas flows through the gas resistor 242 to generate the control pressure on the expiratory valve 241, and during the expiratory stage, the control on the pressure of the patient-exhaled gas can be achieved.

FIG. 2 is schematic structural diagram II of an anesthesia respiration device provided by an embodiment of the disclosure. As shown in FIG. 2, the pressurizing component 311 is a turbine, and the pressure-controlled gas supply branch 32 comprises: a gas source interface 322 and an electromagnetic proportional valve 323 which are connected in sequence. The turbine as the pressurizing component 311 can suck the gas in the atmosphere to generate the driving gas, the gas source interface 322 of the pressure-controlled gas supply branch 32 is connected to a high-pressure gas source, and the electromagnetic proportional valve 323 controls and provides the gas flow of the pressure-controlled gas flowing through the gas resistor 242.

In another embodiment, a third type of pressure-controlled gas supply branch 32 is connected between the pressurizing component 311 and the expiratory pressure control branch 24, and the pressurizing component 311 provides the pressure-controlled gas to the expiratory pressure control branch 24 through the pressure-controlled gas supply branch 32.

Further, the pressure-controlled gas supply branch 32 may further comprise:
a pressure sensor 324 connected between the pressurizing component 311 and the expiratory pressure control branch 24.

Specifically, in the embodiment of the disclosure, when the third type of pressure-controlled gas supply branch 32 is configured, the drive branch 31 further comprises: a switch valve 314.

The switch valve 314 is connected between the pressurizing component 311 and the reflecting component 22.

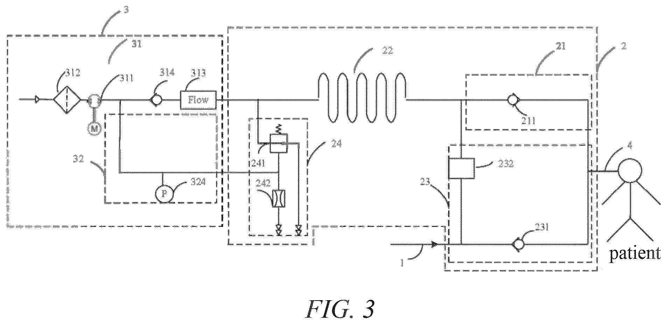

FIG. 3 is schematic structural diagram III of an anesthesia respiration device provided by an embodiment of the disclosure. As shown in FIG. 3, the pressure sensor 324 is connected between the pressurizing component 311 and the expiratory pressure control branch 24, and the switch valve 314 is connected between the pressurizing component 311 and the reflecting component 22. The switch valve 314 may also be a one-way valve, and the specific switch valve is not limited in the embodiment of the disclosure.

It should be noted that in the embodiment of the disclosure, for the third type of pressure-controlled gas supply branch 32, the function of generating both of the driving gas and the pressure-controlled gas is achieved by means of the pressurizing component 311, which can not only control transmission of patient-exhaled gas in the internal loop circuit 2 to the patient, but also control the pressure of the patient-exhaled gas during the expiratory stage.

Specifically, in the embodiment of the disclosure, as shown in FIG. 3, during the inspiratory stage, the pressurizing component 311, i.e., a turbine, can adjust the rotation speed to a higher value so as to output the gas at a higher flow, and output the gas divided into two parts. Part of gas flows through the gas resistor 242 and is discharged into the atmosphere, so as to generate a valve sealing pressure on the expiratory valve 241, and the specific pressure can be detected in real time by means of the pressure sensor 324. The other part of gas flows through the switch valve 314, i.e., a one-way valve, and then flows to the reflecting component 22, and serves as the driving gas to act on the reflecting component 22 and push the patient-exhaled gas stored inside the reflecting component 22 to transfer to the patient through the inspiratory branch 21. At the same time, the fresh gas input through the fresh gas input 1 is also transferred to the patient through the inspiratory branch 21. It should be noted that when the valve sealing pressure needs to be reduced, the rotation speed of the pressurizing component 311, i.e., a turbine, can also be gradually reduced, and then the valve sealing pressure that is generated against the expiratory valve 241 becomes smaller accordingly.

Specifically, in the embodiment of the disclosure, as shown in FIG. 3, during the expiratory stage, the patient-exhaled gas is discharged from the expiratory branch 21, the pressure on the right side of the one-way valve is greater than the pressure on the left side, and the one-way valve is not turned on. The pressurizing component 311 is controlled to output a smaller flow of pressure-controlled gas to the gas resistor 242, i.e., the rotation speed of the turbine is reduced, so as to control the pressure of the patient-exhaled gas discharged through the expiratory valve 241. Of course, it is also possible to directly turn off the pressurizing component 311, i.e., a turbine. In this case, no gas flows through the gas resistor 242, i.e., the control pressure applied to the expiratory valve 241 is zero, and the expiratory valve 241 is turned on, and the pressure control on the patient-exhaled gas is not performed.

An embodiment of the disclosure provides an anesthesia respiration device, comprising: a fresh gas input for inputting a fresh gas; an internal loop circuit for storing a patient-exhaled gas and providing the fresh gas and the patient-exhaled gas to the patient; and an external control branch for controlling the internal loop circuit; wherein the external control branch comprises a drive branch, which comprises: a pressurizing component for controlling transmission of the patient-exhaled gas in the internal loop circuit to the patient; and the fresh gas input and the external control branch are separately connected to the internal loop circuit. That is to say, in the anesthesia respiration device provided by the embodiment of the disclosure, the pressurizing component for controlling transmission of the patient-exhaled gas, stored inside the internal loop, to the patient is disposed in the external control branch outside the internal loop circuit to prevent the pressurizing component from direct contact with the patient-exhaled gas and other impurities, so as to ensure the working capability and accuracy of the pressurizing component.

It should be noted that in the embodiment of the disclosure, the internal loop circuit 2 comprises: an expiratory branch 21, a reflecting component 22, an inspiratory branch 23 and an expiratory pressure control branch 24. In the reflecting component 22, the patient-exhaled gas previously exhaled by the patient is stored. Therefore, when the driving gas is transferred to the internal loop circuit 2, specifically transferred into the reflecting component 2, the patient-exhaled gas is drived to flow through the inspiratory branch 23 and then be transferred to the patient.

It can be understood that in the embodiment of the disclosure, the inspiratory branch 23 comprises an inspiratory one-way valve 231 and an absorption component 232 for absorbing carbon dioxide, wherein the absorption component 232 can absorb the carbon dioxide in the patient-exhaled gas, output re-expiratory gas that can be inhaled again by the patient, and allow the re-expiratory gas to flow through the inspiratory one-way valve 231 and then be supplied to the patient.

It should be noted that in the embodiment of the disclosure, after the step in which the anesthesia respiration device transfers the driving gas to the internal loop circuit to drive and transfer, to the patient, the patient-exhaled gas stored in the internal loop circuit together with the fresh gas that is input to the internal loop circuit through the fresh gas input, the patient-exhaled gas can be received again, and the patient-exhaled gas is stored, comprising: receiving, through the internal loop circuit 2, the patient-exhaled gas output from a patient interface 4; and transferring the patient-exhaled gas to the internal loop circuit 2, and storing the patient-exhaled gas in the internal loop circuit 2.

In the embodiment of the disclosure, during the expiratory stage, the anesthesia respiration device can receive, through the internal loop circuit 2, the patient-exhaled gas output from the patient interface 4.

Specifically, in the embodiment of the disclosure, during the expiratory stage, the expiratory branch 21 in the internal loop circuit 2 can receive the patient-exhaled gas from the patient interface 4.

It can be understood that in the embodiment of the disclosure, during the inspiratory stage, the patient-exhaled gas can be subjected to certain absorption treatment and then be repeatedly transferred to the patient, i.e., for recycling. Therefore, during the expiratory stage, the patient-exhaled gas can be received and repeatedly supplied to the patient again in the next inspiratory stage.

In the embodiment of the disclosure, after the anesthesia respiration device receives, through the internal loop circuit 2, the patient-exhaled gas output from the patient interface 4, the patient-exhaled gas can be stored in the internal loop circuit 2.

Specifically, in the embodiment of the disclosure, in the anesthesia respiration device, the expiratory branch 21 receiving the patient-exhaled gas in the internal loop circuit 2 is connected to the reflecting component 22, and the patient-exhaled gas will be transferred to the reflecting component 22, and specifically stored in the reflecting component 22.

It should be noted that in the embodiment of the disclosure, during the expiratory stage, the anesthesia respiration device may also achieve the control on the pressure of the patient-exhaled gas by means of the external control branch 3.

Specifically, in the embodiment of the disclosure, the anesthesia respiration device needs to firstly generate a control pressure on the internal loop circuit 2 by means of the external control branch 3, comprising: acquiring a pressure-controlled gas through the external control branch 3; transferring the pressure-controlled gas from the external control branch 3 to internal loop circuit 2; and generating the control pressure on the internal loop circuit 2 when the pressure-controlled gas flows through the internal loop circuit 2.

It can be understood that in the embodiment of the disclosure, the external control branch 3 comprises a drive branch 31 and a pressure-controlled gas supply branch 32. The pressure-controlled gas supply branch 32 has three modes, which have been described in detail in the above embodiments and will not be repeated here. The anesthesia respiration device can obtain the pressure-controlled gas through the pressure-controlled gas supply branch 32 of the external control branch 3, and the pressure-controlled gas will be transferred into the internal loop circuit 2, and will specifically flow through the gas resistor 242 of the expiratory pressure control branch 24 in the internal loop circuit 2, so as to generate the control pressure on the expiratory valve 241 connected to the gas resistor 242.

It should be noted that in the embodiment of the disclosure, in the anesthesia respiration device, the pressure-controlled gas generates the control pressure on the internal loop circuit 2, i.e., when the control pressure is generated at the expiratory valve 241, during the expiratory stage, the control on the pressure of the patient-exhaled gas can be achieved.

It can be understood that in the embodiment of the disclosure, during the expiratory stage, when the anesthesia respiration device stores the patient-exhaled gas in the reflecting component 22, the driving gas previously transferred during the inspiratory stage is stored in the reflecting component 22, at this time there is a control pressure at the expiratory valve 241, and when the patient-exhaled gas is greater than the control pressure, i.e., the pressure of the patient-exhaled gas is sufficient to remove the control pressure at the expiratory valve 241 to push open the valve gate of the expiratory valve 241, so as to discharge the driving gas in the reflecting component 22 from the expiratory valve 241.

It should be noted that in the embodiment of the disclosure, the anesthesia respiration device may also not perform control on the pressure of the patient-exhaled gas, i.e., without supplying the pressure-controlled gas through the pressure-controlled gas supply branch 32 of the external control branch 3. Therefore, during the expiratory stage, no control pressure is generated at the expiratory valve 241, and all the driving gas can be directly discharged.

An embodiment of the disclosure provides an anesthesia respiration method (not claimed) applied to an anesthesia respiration device. The anesthesia respiration device sucks the gas in the atmosphere by means of the pressurizing component of the drive branch in the external control branch to generate a driving gas. The driving gas is transferred to the internal loop circuit to drive and transfer, to the patient, the patient-exhaled gas stored in the internal loop circuit together with the fresh gas that is input to the internal loop circuit through the fresh gas input. That is to say, in the anesthesia respiration method provided by the embodiment of the disclosure, the driving gas can be generated by means of the pressurizing component disposed in the external control branch to control transmission of the patient-exhaled gas stored in the internal loop circuit to the patient to prevent the pressurizing component from direct contact with the patient-exhaled gas and other impurities, so as to ensure the working capability and accuracy of the pressurizing component.

The above description is only preferred embodiments of the disclosure, and is not intended to limit the scope of protection of the disclosure.

### Industrial applicability

The anesthesia respiration device of an embodiment of the disclosure comprises: a fresh gas input for inputting a fresh gas; an internal loop circuit for storing a patient-exhaled gas and providing the fresh gas and the patient-exhaled gas to the patient; and an external control branch for controlling the internal loop circuit; wherein the external control branch comprises a drive branch, which comprises: a pressurizing component for controlling transmission of the patient-exhaled gas in the internal loop circuit to the patient; and the fresh gas input and the external control branch are separately connected to the internal loop circuit. That is to say, in the anesthesia respiration device provided by the embodiment of the disclosure, the pressurizing component for controlling transmission of the patient-exhaled gas, stored in the internal loop circuit, to the patient is disposed in the external control branch outside the internal loop circuit to prevent the pressurizing component from direct contact with the patient-exhaled gas and other impurities, so as to ensure the working capability and accuracy of the pressurizing component.

The invention is defined by the following claims:

## Claims

1. An anesthesia respiration device, wherein the anesthesia respiration device comprises:
a fresh gas input (1) for inputting a fresh gas;
an internal loop circuit (2) for storing a patient-exhaled gas and providing the fresh gas and the patient-exhaled gas to the patient; wherein
the internal loop circuit (2) comprises:
- an expiratory branch (21) for receiving the patient-exhaled gas through a patient interface (4);
- a reflecting component (22) for storing the patient-exhaled gas;
- an inspiratory branch (23) for transferring the fresh gas and the patient-exhaled gas that is stored in the reflecting component (22) to the patient through the patient interface (4), wherein; the inspiratory branch (23) is connected to the reflecting component (22) and the fresh gas input (1);
- an expiratory pressure control branch (24) for controlling a pressure of the patient-exhaled gas, wherein the expiratory pressure control branch (24) is connected to the reflecting component (22); and
an external control branch (3) for controlling the internal loop circuit (2);
wherein the external control branch (3) comprises a drive branch (31), wherein the drive branch (31) comprises
a pressurizing component (311) for controlling transmission of the patient-exhaled gas in the internal loop circuit (2) to the patient; and wherein
the reflecting component (22) is connected to the expiratory branch (21) and the drive branch (31), and the fresh gas input (1) and the external control branch (3) are separately connected to the internal loop circuit (2);
**characterized in that** the external control branch (3) further comprises
a pressure-controlled gas supply branch (32), connected to the expiratory pressure control branch (24), for providing a pressure-controlled gas, and controlling the expiratory pressure control branch (24) to perform pressure control on the patient-exhaled gas.

2. The device of claim 1, wherein the pressurizing component (311) is any one of a turbine, a pressurizing pump, an air compressor and a cylinder.

3. The device of claim 1, wherein drive branch (31) further comprises at least one of a filter (312) and a flow sensor (313); wherein
an outlet of the filter (312) is connected to the pressurizing component (311); and
the flow sensor (313) is connected between the pressurizing component (311) and the internal loop circuit (2).

4. The device of claim 1, wherein the pressure-controlled gas supply branch (32) comprises
a pressure control component (321) connected to the expiratory pressure control branch (24).

5. The device of claim 4, wherein the pressure control component (321) is any one of a turbine, a pressurizing pump, an air compressor and a cylinder.

6. The device of claim 4, wherein the pressure-controlled gas supply branch (32) comprises a gas source interface (322) and an electromagnetic proportional valve (323) which are connected in sequence;
wherein an outlet of the electromagnetic proportional valve (323) is connected to the expiratory pressure control branch (24).

7. The device of claim 1, wherein the pressure-controlled gas supply branch (32) is connected between the pressurizing component (321) and the expiratory pressure control branch (24).

8. The device of claim 7, wherein the pressure-controlled gas supply branch (32) comprises
a pressure sensor (324) connected between the pressurizing component (321) and the expiratory pressure control branch (24).

9. The device of claim 7, wherein the drive branch (3) further comprises
a switch valve (314) connected between the pressurizing component (321) and the reflecting component (22).

10. The device of claim 1, wherein the expiratory pressure control branch (24) comprises an expiratory valve (241) and a gas resistor (242);
wherein an inlet of the expiratory valve (241) is connected to the reflecting component (22); and
the gas resistor (242) is connected to a valve gate of the expiratory valve (241).

## Patentansprüche

1. Ein Anästhesie-Beatmungsgerät, wobei das Anästhesie-Beatmungsgerät Folgendes umfasst:
einen Frischgaseingang (1) zum Einspeisen vom Frischgas;
einen internen Kreislauf (2) zum Speichern eines vom Patienten ausgeatmeten Gases und zur Bereitstellung des Frischgases und des vom Patienten ausgeatmeten Gases für den Patienten; wobei der interne Kreislauf (2)
Folgendes umfasst:
- einen Exspirationszweig (21) zur Aufnahme des vom Patienten ausgeatmeten Gases über eine Patientenschnittstelle (4);
- eine reflektierende Komponente (22) zum Speichern des vom Patienten ausgeatmeten Gases;
- einen Inspirationszweig (23) zum Übertragen des Frischgases und des vom Patienten ausgeatmeten Gases, das in der reflektierenden Komponente (22) gespeichert ist, über die Patientenschnittstelle (4) zum Patienten, wobei
; der Inspirationszweig (23) mit der reflektierenden Komponente (22) und dem Frischgaseingang (1) verbunden ist;
- einen Steuerzweig für den Exspirationsdruck (24) zur Steuerung des Drucks des vom Patienten ausgeatmeten Gases, wobei der Steuerzweig für den
Exspirationsdruck (24) mit der reflektierenden Komponente (22) verbunden ist; und einen externen Steuerzweig (3) zur Steuerung des internen Regelkreises (2); wobei der externe Steuerzweig (3) einen Antriebszweig (31) umfasst, wobei der Antriebszweig (31)
eine Druckkomponente (311) zur Steuerung der Übertragung des
vom Patienten ausgeatmeten Gas im internen Kreislauf (2) zum Patienten umfasst; und wobei
die reflektierende Komponente (22) mit dem Exspirationszweig (21) und dem Antriebszweig (31) verbunden ist und der Frischgaseingang (1) und der externe Steuerzweig (3) separat mit dem internen Kreislauf (2) verbunden sind;
**dadurch gekennzeichnet, dass** der externe Steuerzweig (3) weiterhin Folgendes umfasst:
einen Versorgungszweig (32) für druckgesteuertes Gas, der mit dem Steuerzweig (24) für den Exspirationsdruck verbunden ist, um ein druckgesteuertes Gas bereitzustellen, und um den Steuerzweig (24) für den Exspirationsdruck zu steuern, um eine Drucksteuerung für das vom Patienten ausgeatmete Gas durchzuführen.

2. Vorrichtung nach Anspruch 1, wobei die Druckkomponente (311) eine Turbine, eine Druckpumpe, ein Luftkompressor oder ein Zylinder ist.

3. Vorrichtung nach Anspruch 1, wobei der Antriebszweig (31) außerdem mindestens
einen Filter (312) und einen Durchflusssensor (313) umfasst; wobei
ein Auslass des Filters (312) mit der Druckkomponente (311) verbunden ist; und
der Durchflusssensor (313) zwischen der Druckkomponente (311) und dem internen Kreislauf (2) angeschlossen ist.

4. Vorrichtung nach Anspruch 1, wobei der Versorgungszweig für druckgesteuertes Gas (32)
eine Drucksteuerkomponente (321) umfasst, die mit dem Steuerzweig für den Exspirationsdruck (24) verbunden ist.

5. Vorrichtung nach Anspruch 4, wobei die Drucksteuerkomponente (321) eine Turbine, eine Druckpumpe, ein Luftkompressor oder ein Zylinder ist.

6. Vorrichtung nach Anspruch 4, wobei der Versorgungszweig für druckgesteuertes Gas (32) eine Gasquellenschnittstelle (322) und ein elektromagnetisches Proportionalventil (323) umfasst, die nacheinander verbunden sind; wobei ein Ausgang des elektromagnetischen Proportionalventils (323) mit dem Steuerzweig für den Exspirationsdruck (24) verbunden ist.

7. Vorrichtung nach Anspruch 1, wobei der Versorgungszweig für druckgesteuertes Gas (32) zwischen der Drucksteuerkomponente (321) und dem Steuerzweig für den Exspirationsdruck (24) angeschlossen ist.

8. Vorrichtung nach Anspruch 7, wobei der Versorgungszweig für druckgesteuertes Gas (32) Folgendes umfasst:
einen Drucksensor (324), der zwischen der Drucksteuerkomponente (321) und dem Steuerzweig für den Exspirationsdruck (24) angeschlossen ist.

9. Vorrichtung nach Anspruch 7, wobei der Antriebszweig (3) weiterhin Folgendes umfasst:
ein Schaltventil (314), das zwischen der Drucksteuerkomponente (321) und der reflektierenden Komponente (22) angeschlossen ist.

10. Vorrichtung nach Anspruch 1, wobei der Steuerzweig für den
Exspirationsdruck (24) ein Exspirationsventil (241) und einen Gaswiderstand (242) umfasst: wobei ein Einlass des Exspirationsventils (241) mit der reflektierenden Komponente (22) verbunden ist; und der Gaswiderstand (242) mit einem Ventilverschluss des Exspirationsventils (241) verbunden ist.

## Revendications

1. Dispositif de respiration d'anesthésie, où le dispositif de respiration d'anesthésie comprend :
une entrée de gaz frais (1) destinée à faire entrer un gaz frais ;
un circuit de boucle interne (2) destiné à stocker un gaz exhalé par un patient et à fournir le gaz frais et le gaz exhalé par le patient au patient, où le circuit de boucle interne (2) comprend :
- une branche expiratoire (21) destinée à recevoir le gaz exhalé par le patient par l'intermédiaire d'une interface patient (4) ;
- un composant de réflexion (22) destiné à stocker le gaz exhalé par le patient ;
- une branche inspiratoire (23) destinée à transférer le gaz frais et le gaz exhalé par le patient qui est stocké dans le composant de réflexion (22) au patient par l'intermédiaire de l'interface patient (4), où le branche inspiratoire (23) est reliée au composant de réflexion (22) et à l'entrée de gaz frais (1) ;
- une branche de contrôle de pression expiratoire (24) destinée à contrôler une pression du gaz exhalé par le patient, où la branche de contrôle de pression expiratoire (24) est reliée au composant de réflexion (22) ; et
une branche de contrôle externe (3) destinée à contrôler le circuit de boucle interne (2) ;
où la branche de contrôle externe (3) comprend une branche d'entraînement (31), où la branche d'entraînement (31) comprend
un composant de mise en pression (311) destiné à contrôler une transmission du gaz exhalé par le patient dans le circuit de boucle interne (2) au patient ; et où
le composant de réflexion (22) est relié à la branche expiratoire (21) et à la branche d'entraînement (31), et l'entrée de gaz frais (1) et la branche de contrôle externe (3) sont séparément reliées au circuit de boucle interne (2) ;
**caractérisé en ce que** la branche de contrôle externe (3) comprend en outre
une branche d'alimentation en gaz contrôlé en pression (32), reliée à la branche de contrôle de pression expiratoire (24), destinée à fournir un gaz contrôlé en pression, et à contrôler la branche de contrôle de pression expiratoire (24) afin de réaliser un contrôle de pression sur le gaz exhalé par le patient.

2. Dispositif selon la revendication 1, dans lequel le composant de mise en pression (311) est un élément quelconque parmi une turbine, une pompe de mise en pression, un compresseur d'air et un vérin.

3. Dispositif selon la revendication 1, dans lequel la branche d'entraînement (31) comprend en outre au moins un élément parmi un filtre (312) et un capteur de débit (313) ; où
une sortie du filtre (312) est reliée au composant de mise en pression (311) ; et
le capteur de débit (313) est relié entre le composant de mise en pression (311) et le circuit de boucle interne (2).

4. Dispositif selon la revendication 1, dans lequel la branche d'alimentation en gaz contrôlé en pression (32) comprend
un composant de contrôle de pression (321) relié à la branche de contrôle de pression expiratoire (24).

5. Dispositif selon la revendication 4, dans lequel le composant de contrôle de pression (321) est un élément quelconque parmi une turbine, une pompe de mise en pression, un compresseur d'air et un vérin.

6. Dispositif selon la revendication 4, dans lequel la branche d'alimentation en gaz contrôlé en pression (32) comprend une interface source de gaz (322) et une électrovanne proportionnelle (323) qui sont reliées en série ;
où une sortie de l'électrovanne proportionnelle (323) est reliée à la branche de contrôle de pression expiratoire (24).

7. Dispositif selon la revendication 1, dans lequel la branche d'alimentation en gaz contrôlé en pression (32) est reliée entre le composant de mise en pression (321) et la branche de contrôle de pression expiratoire (24).

8. Dispositif selon la revendication 7, dans lequel la branche d'alimentation en gaz contrôlé en pression (32) comprend
un capteur de pression (324) relié entre le composant de mise en pression (321) et la branche de contrôle de pression expiratoire (24).

9. Dispositif selon la revendication 7, dans lequel la branche d'entraînement (3) comprend en outre
un vanne de commutation (314) reliée entre le composant de mise en pression (321) et le composant de réflexion (22).

10. Dispositif selon la revendication 1, dans lequel la branche de contrôle de pression expiratoire (24) comprend une vanne expiratoire (241) et un élément de résistance au gaz (242) ;
où une entrée de la vanne expiratoire (241) est reliée au composant de réflexion (22) ; et
l'élément de résistance au gaz (242) est relié à un corps de vanne de la vanne expiratoire (241).
